# EUROPEAN PATENT APPLICATION

(11) **EP 3 153 840 A1**
(43) Date of publication of application: **12.04.2017**
(21) Application number: 14894285.7
(22) Date of filing: 24.09.2014
(51) Int. Cl.: G01N 7/18, G01N 33/50

(54) **QUANTITATIVE DETECTION METHOD FOR TARGET BASED ON AIR PRESSURE DETECTION**

(30) Priority: 09.06.2014 CN 201410252334
(71) Applicant: Xiamen University, Xiamen, Fujian 361000 (CN)
(72) Inventor: YANG, Chaoyong, Xiamen Fujian 361000 (CN); GUAN, Zhichao, Xiamen Fujian 361000 (CN); JIA, Shasha, Xiamen Fujian 361000 (CN); ZHU, Zhi, Xiamen Fujian 361000 (CN); LIU, Dan, Xiamen Fujian 361000 (CN); ZHANG, Mingxia, Xiamen Fujian 361000 (CN); LIN, Shuichao, Xiamen Fujian 361000 (CN); LI, Jiuxing, Xiamen Fujian 361000 (CN); ZHUANG, Zhixia, Xiamen Fujian 361000 (CN)
(74) Representative: Verscht, Thomas Kurt Albert
(86) International application number: PCT/CN2014/087243
(87) International publication number: WO 2015/188512

(57) **Abstract**

Disclosed is a quantitative analysis method based on air pressure detection, which can be used for the high-sensitivity quantitative detection of various targets having inorganic ions, micromolecules and biological macromolecules such as proteins, DNA, and even viruses, bacteria, cells, etc. The present invention catalyzes hydrogen peroxide to generate a large amount of gas using enzymes or nanometer particles, etc.; converts a target molecule detection signal into a gas pressure intensity signal; achieves signal amplification; and finally converts the pressure intensity change into an electrical signal to conduct a reading through a barometer, thereby achieving high-sensitivity quantitative detection. In the present invention, the feasibility, wide applicability and reliability of the present invention are certified through three different detection systems, i.e. an ELISA system, a DNA hydrogel and a functional DNA sensor, respectively, using a barometer.

## Description

### Field of the invention

The present invention is related to a quantitative analysis method based on air pressure measuring, which can be used for high-sensitivity quantitative detection of various targets i.e. inorganic ions, small molecules and biological macromolecules such as proteins, DNA, and even viruses, bacteria, cells, etc.

### Background of the invention

Developing novel, sensitive, highly specific, cheap and convenient quantitative analysis methods are one of the hottest topic in the scientific research work always. Existing methods of quantitative detection and analysis usually depended on all kinds of bulky and precise instruments, such as spectrometer, mass spectrometry, chromatography, electrophoresis, etc. These quantitative analysis methods had been well developed for a long time, and mature detection methods and techniques had been established, therefore they are widely used in sample testing and analysis in the fields of biology, chemistry, medicine, food and so on, such as various kinds of organic small molecules and biological macromolecules and bacteria, viruses, cell, etc. However, whether the mass spectrometry, chromatography, electrophoresis, or the optical analysis instruments, they have high demands on the sensitivity of detection components and on the precision of instrument to realize high sensitive detection and quantitative analysis, so that the cost of the detecting instruments and the cost of detecting experiments are hard to decrease.

For example, quantitative instruments based on optics, such as common ultraviolet-visible spectrophotometer, UV spectrometer, fluorescence spectrometer, etc. In order to realize the precise quantitative detection analysis with high sensitivity, these instruments not only needed to be provided complex and sophisticated light route components, i.e. grating, filter, lens group, they are also needing precise control by experienced operators. In addition, to improving the sensitivity of these instruments, high power light sources are essential to enrich and amplify optical signal. At the same time, detecting methods based on optics have high requirements for detecting system. On the one hand, the components of these detection systems cannot be too complicated, when the molecules or material of non-target had similar optical properties with the target molecules, they will seriously affect the accuracy of detection, therefor the optical detection system usually require complex pretreatment steps to avoid the aforementioned problem; on the other hand, Not all molecules have good optical properties, thus molecular recognition technics are widely used for molecular marker and signal amplification, enzyme-linked immunoassay, for example, is to generate product with fluorescent properties or optical absorption properties by the use of immunological methods, to achieve the detection of target antigen (1, Engvall E, Perlmann P. Enzyme - linked immunosorbent assay (ELISA) policy assay of immunoglobulin G. Immunochemistry [J], 1971, 8 (9) : 871-874.). The above problems make the cost for optical quantitative analysis hard to be reduced further. Therefore, how to develop a quantitative detection method with low cost, affordable but have good sensitivity is increasingly attracting the attention of the researchers.

At the same time, with the development of science and technology, and improvement of the recognition of science and life, people no longer content with the costly and time consuming laboratory experiment testing, they are more hope that the science can be combined with the application of life, to realize "technology changes life". Such as the concept of "instant diagnosis" has attracted more and more attention (2, r. j. Davies, s. s. e., s. j. Carlisle, faced of Biosensors and Biochips, Wiley, Hoboken [M]. 2008. A; 3, Liu, H., Xiang, Y., Lu, Y., Mr Crooks, r. m., Aptamer - -based origami paper analytical device for electrochemical detection of adenosine, Angewandte Chemie - International Edition [J]. 2012, 51, 6925-8.; 4, Liu, j. w., Oligonucleotide - functionalized hydrogels as stimuli responsive materials and Biosensors, Soft Matter [J]. 2011, 7. 6757-6767.). Said instant diagnosis (Point of Care Test, POC Test) refers to process real-time detection and diagnosis to realize the rapid monitoring and treatment in the position where the disease occurred or the events happened. Modern POC test methods often use immune analysis technology, the combination of specific antibody and antigen, to capture the target and analyzed. The disease specific target protein markers are used as detecting target, to realize the diagnosis of diseases, for example, the pregnancy test kit use the pregnancy hormone human chorionic gonadotropin (hCG) as the target. However, without the support of the precision instruments, POC detection sensitivity will surely be affected. Existing POC testing generally can only realize the qualitative or half-quantitative detection, so that they cannot be achieved for the accurate diagnosis of disease or condition. So although POC detection can provide certain information in real-time diagnosis and emergency response, it cannot provide more accurate basis for more rapid clinical treatment. Establishing a miniaturized, affordable and fast detection method with high sensitivity is extremely urgent.

In conclusion, in scientific research of the future, how to develop a kind of quantitative analysis method, which does not affect the advantages of high sensitivity, high selectivity, high accuracy of experimental method, and can also significantly reduce the cost of testing instrument and the experiment, not only can realize laboratory experiment requirements of high sensitivity, high accuracy, but also has the advantages of miniaturization, portable and low energy consumption, and can be applied in the instant diagnosis, is urgently to be solved.

### Summary of the invention

According to the disadvantages of existing quantitative test methods such as expensive, high cost of experiment in existing quantitative test methods, the present invention, based on air pressure measuring, developed a kind of quantitative analysis method with high sensitivity, high selectivity, high accuracy, affordability and portability. The method not only can be used in laboratory, to detect inorganic ions, small molecules and biological macromolecules such as proteins, DNA, and even viruses, bacteria, cell and other targets with high sensitivity and high selectivity, but also can be used for rapid analysis of quantitative POC detection with high sensitivity.

The object of the invention is achieved by:
A quantitative, versatile targets analysis method with high sensitivity based on air pressure measuring, is by introducing signal amplification molecules such as enzymes or nanoparticles into an air-sealed system, and release a large amount of gas molecules is generated by the action of the molecular signal amplification molecules or particles catalyze the substrate, cause the system pressure increasing. measure the system pressure changes of the reacting detection system to detect the target concentration.

Wherein, said molecular recognition technology is that, identifying or labeling targets by using specific molecular with recognizing function, such as antibody, aptamer, nucleic acid probe etc., and then the signal molecules or particles are introduced into the detection system through the recognition molecular.

Wherein, in the target recognizing or labeling by molecular recognition technology, said target including but not limited to proteins, nucleic acids, peptides, sugars, lipids, small organic molecules, inorganic ions, cells, bacteria and viruses.

Wherein, said signal amplification molecules comprising catalase, gold nanoparticles, platinum nanoparticles, gold platinum nanoparticles, manganese oxide nanoparticles or other enzyme or catalysts able to catalyze substrate to produce the gas, the catalytic substrate is the material which can produce a large amount of gas molecules (H202) after being catalyzed, gas molecules are the product (e.g., 02) after the substrate being catalyzed.

Wherein, in the air-sealed system, the reaction detection system comprising enzyme-linked immunosorbent assay(ELISA), DNA hydrogel system, or functional DNA sensing system.

In enzyme-linked immunosorbent assay system, quantitative analysis method based on air pressure measuring comprises the following steps: (1) select corresponding capture antibodies and detection antibodies according to the testing antigens; (2) modify the signal amplification molecules, label them with detection antibody molecules, or able to specifically conjugate to detection antibodies; (3) coat the solid surface which can be used for enzyme-linked immunosorbent assay such as porous plate (for example. 96 - well plates) or magnetic beads or microspheres, add capture antibodies to let they combine on the solid surface after the coating, and then block the surface with blocking solution, add the antigen to be detected, and add detection antibody, form double-antibody sandwich structure, wash away excess detection antibodies; (4) introduce signal amplification molecules; (5)add substrate in the perforated plate, seal the reaction cavities, signal amplification catalyze the substrate molecules, then a large amount of gas molecules are generated, and generate the pressure variation signal which can be read by air pressure meter; (6) the target molecule concentration is quantitatively measured according to the pressure value changes of the air pressure meter.

Wherein, the perforated plate surface can be used as the solid surface for capturing antibody, to capture target molecules; also, magnetic beads or microspheres can be used as the solid surface for capturing antibody, to realize the capture of target molecules by methods such as magnetic field or centrifugation.

Wherein, the introducing method of the signal amplification molecules or nanoparticles can be the conjugating for detection antibodies directly, or can be the specific molecules for recognizing the detected antibody modified to the signal amplification molecules or nanoparticles, after detecting antibodies and target molecules combined, the signal molecules or nanoparticles are conjugated to the detection antibodies, allows the signal amplification molecules or nanoparticles has better versatility and applicability.

In DNA hydrogel system, quantitative analysis method for multiple targets based on air pressure measuring in real-time visualization of optimization preferredly comprises the following steps: (1) mix two poly-acrylamide-DNA strands, an aptamer and enzyme molecules or nanoparticles, to prepare DNA aptamer crosslinked hydrogel; (2) add solution containing different known concentrations of analyte to the hydrogel, releasing enzymes or nanoparticles; (3) after the reaction, the reaction supernatant is put into a reaction tube, and make the enzyme or nano particles and substrate process catalytic reaction; in a air-sealed system, the large amount of gas make the pressure rise inside the system, use air pressure meter to read, and record the data, then standard curve can be established, thus the content of target in the unknown samples can be detected.

Wherein, DNA hydrogel is formed by two poly-acrylamide-DNA strands, and an aptamer whose both ends can respectively complementary to the DNA sequence of the two poly strands.

Wherein, the enzyme molecules or nano particles are packaged in the cavities of three-DNA-strands structure of the DNA hydrogel, by molecule steric hindrance, the enzyme molecules or nano particles are restricted and cannot be diffused freely. When target molecules exist, the structure of aptamer will be changed, cause the DNA hydrogel collapse, and release the enzyme molecules or nanoparticles.

In functional DNA sensing system, a visualized-quantitative POCT method based on air pressure measuring for multiple targets, prefferedly comprises the following steps: (1) select appropriate aptamer according to the target molecules, add a sequence complementary to capture probe to the aptamer; (2) design suitable detection probe sequence according to the sequence of aptamer, allow when there are not target molecular, the detection probe can combine on the aptamer strand through the interaction between bases, while when the target molecule exist, the detection probes can be replaced by the target molecule; (3) connect the capture probe onto the surface of magnetic bead by biological or chemical conjugation method, such as the decarboxylation reaction or biotin avidin interaction, add detection DNA and aptamer, form sandwich hybridization structure of functional ization DNA on the surface of the magnetic beads; (4) add target in DNA sandwich hybridization system , target combined to aptamer, thus the detection probe on three strand structure be replaced out; (5) use magnet to enrich magnetic beads, and put the supernatant into a reaction tube, add a solution phase substrates, signal amplification molecules act with solution phase substrates, then produce signal molecules which can be read by the air pressure meter. (6) record test results according to air pressure meter readings.

Wherein, the detection probe conjugated with signal amplification molecules, such as enzyme or nanoparticles, which can catalyze substrate to generate a large amount of gas.

Wherein, the combination of the aptamer and the target molecules, enables the target molecules to compete and replace the detection probe conjugated with signal amplification molecules (enzyme or nanoparticles), and realize following signal amplification.

Wherein, the magnetic beads used in step (3) can also be replaced by perforated plate or microspheres, accordingly, the method of removing the detecting probe un-replaced can be centrifugation (microsphere) or directly remove supernatant(porous plate).

Wherein, the detection probe conjugating with the signal amplification (enzyme or nanoparticles) through chemical or biological conjugation methods, introducing enzymes or nanoparticles is by the specificity of detection probe.

The advantages of the invention are: firstly, the developed method matches to the ASSURED international standards on the design (Martinez a., Phillips, s., Whitesides g., et. Al., Diagnostics for the Developing World: Microfluidic Paper - -based Analytical Devices, Analytical Chemistry [J]. 2010, 82. 3-10.), and our method has high sensitivity, good selectivity, and reliable detecting results; Secondly, Au @ PtNPs has the features such as easily being stored, can last a long time of catalyze etc. By extending reaction time between Au @ PtNPs and H2O2, the detection of low-concentration target can be realized, and by transforming the O2 volume into pressure, a quantitative relationship between target concentration and the air pressure meter readings is finally established; In addition, due to the rapid development of the SELEX technology, there are many available DNA aptamers, and more aptamers for a wider range of targets can also be obtained by SELEX selection, in hydrogel and functional DNA sensing system, we only simply need to replace the specific sequences of aptamer, thus our method can be used for rapid, quantitative analysis of more other targets. In addition, in ELISA system, due to the traditional ELISA need to use the mark of HRP enzymes or other enzymes for color reaction, and then to realize quantitative detection to antibody or antigen, this needs to rely on bulky equipment, and means that it must be used in the laboratory or in a hospital, and unable to realize real-time diagnosis. While in this invention, it just only needs simple air pressure meter, then the quantitative detection will be realized, and only needs simply replace the antibody or antigen, different antigen detection can be realized. In consideration of low cost, fast, user friendly and wide availability of air pressure meter, our visualization quantitative analysis method based on pressure detection can become public quantitative detection tools used in a wide variety of targets.

The method of the present invention has high detection sensitivity, and has advantages such as simple operation, low cost, quick response, the samples don't need to be pretreat complexly, etc., and also has good versatility, and can be used for rapid, high sensitive quantitative detection for many substances, comprising biological small molecules, heavy metal ions, proteins tec. in complex system.

### Brief description of the drawings

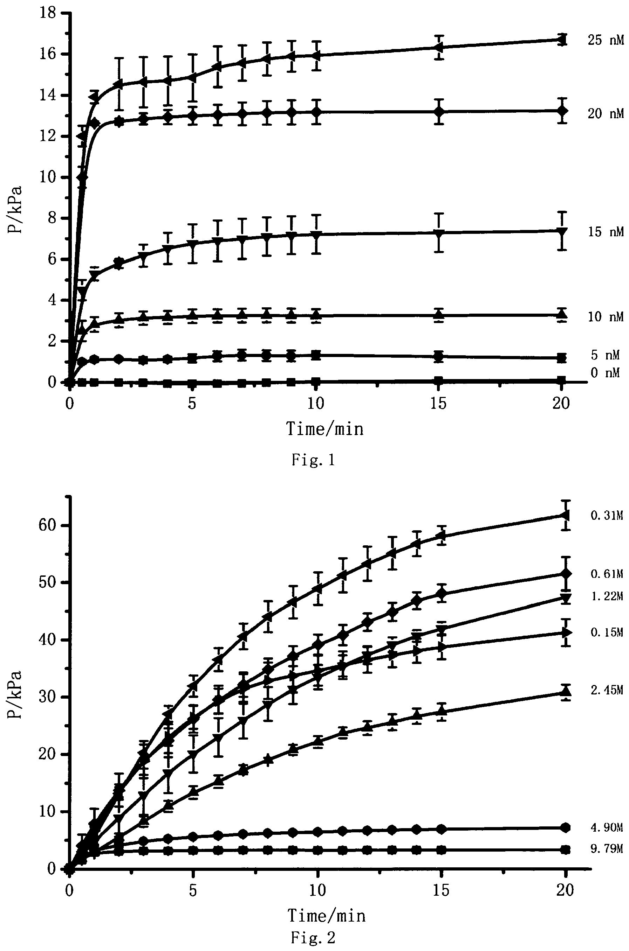

Figure 1, at room temperature, the catalytic efficiency of hydrogen peroxide enzyme (Catalase) of different concentrations under 9.79 M H202.
Figure 2, at room temperature, under the different concentration of H202, the catalytic efficiency of 5 nM hydrogen peroxide enzyme (Catalase).

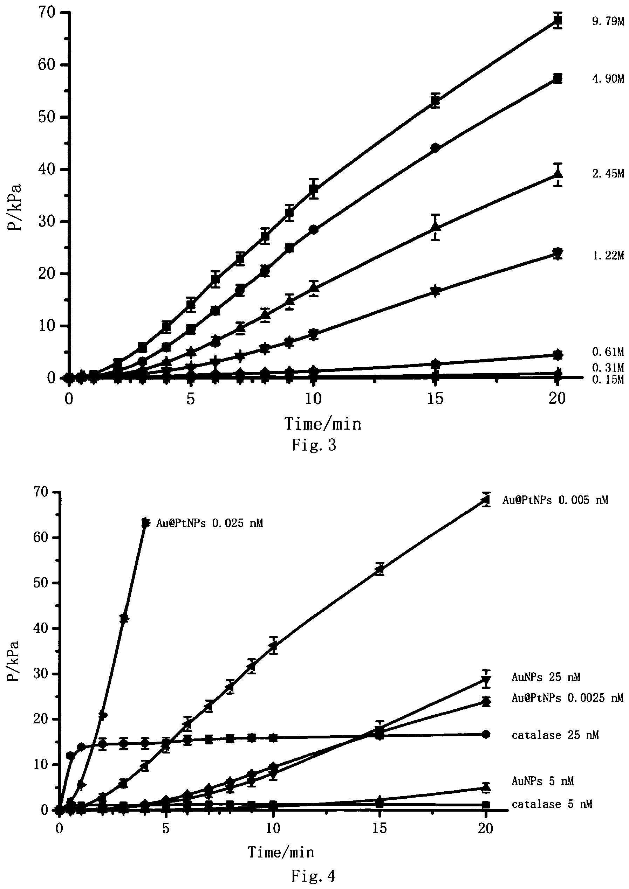

Figure 3, at room temperature, under the different concentration of H202, the catalytic efficiency of 0.005 nM Au @ PtNPs.
Figure 4, at room temperature, the contrast of the catalytic efficiency among hydrogen peroxide enzyme (Catalase), AuNPs, Au @ PtNPs.
Figure 5, at room temperature, by the output mode of pressure, when the H202 concentration is 9.79 M, the catalytic efficiency in 1hr of different concentrations of Au @ PtNPs.
Figure 6, at room temperature,in ELISA system, the signal amplification molecules are biotinylated catalase, in 1 hr, for H5N1 avian influenza virus HA protein ELISA detection, investigation of catalytic activity of hydrogen peroxide enzyme (Catalase).
Figure 7, at room temperature, in ELISA system, the feasibility of detection of recombinant protein system of human prostate specific antigen (PSA) was vitrificated in conventional ELISA method.
Figure 8, at room temperature, in ELISA system, use nanoparticles are used to detect different concentration of target protein, target people detect prostate specific antigen (PSA) recombinant protein.
Figure 9, at room temperature, in ELISA system, human serum albumin (HAS), sheep anti rat (IgG), matrix metalloproteinases (MMPS), thrombin (Thr) are used as negative contrast, investigate the selectivity of prostate specific antigen (PSA) in the system, the figure are the response of 120 um HSA, IgG, MMP, Thr, PSA, the reaction time is 1 hr.
Figure 10, at room temperature, in ELISA system, on the basis of conventional ELISA method, investigate detecting accuracy of nanoparticles used to target protein, prostate specific antigen (PSA) of different concentration.
Figure 11, at room temperature, in ELISA system, feasibility of H5N1 avian flu virus HA protein system of conventional ELISA.
Figure 12, at room temperature, in ELISA system, use nanoparticles to detect target protein, the H5N1 avian flu virus HA protein of different concentrations.
Figure 13, at room temperature, in ELISA system, the goat-anti-mouse second antibody (IgG), human serum albumin (HSA), influenza a (H3N2) virus (H3N2), SARS coronavirus (SARS) are used as negative contrast, investigate the selectivity of the H5N1 avian flu virus HA protein in the system, the figure is the responds about 120 um H5N1, IgG, HSA, H3N2, SARS, the reaction time is 1 hr.
Figure 14, at room temperature, ELISA system, in the serum environment, detect the target protein, the H5N1 avian flu virus HA protein of different concentration by the use of nanoparticles.
Figure 15, at room temperature, ELISA system, feasibility of people matrix metalloproteinases (MMP9) recombinant protein system by conventional ELISA.
Figure 16, at room temperature, ELISA system, the use of nanoparticles, detect target protein, people matrix metalloproteinases (MMP9) of recombinant proteins of different concentrations.
Figure 17, at room temperature, ELISA system, thrombin (Thr) and lysozyme (Lys), sheep-anti- rat (IgG) and human serum albumin (HSA) are used as negative control, investigate the selectivity of matrix metalloproteinases (MMP9) in the system. and the figure shows the response about to 120 µm MMPS, Thr, Lys, IgG, HSA, the reaction time is 1 hr.
Figure 18, at room temperature, in the DNA hydrogel system, detecting for different concentrations of cocaine, the reaction time is 5 min.
Figure 19, at room temperature, in the DNA hydrogel system, detecting for different concentrations of cocaine, the reaction time is 15 min.
Figure 20, at room temperature, in the DNA hydrogel system, in the actual urine sample system, detecting for different concentrations of cocaine, the reaction time is 5 min.
Figure 21, at room temperature, in the DNA hydrogel system, benzoyl ecgonine and methyl ecgonine of cocaine metabolites are used as negative contrast, investigate t the selectivity of the system, the figure shows responses to 250 µm cocaine and 2.5 mM benzoyl ecgonine and methyl ecgonine, the reaction time is 5 min.
Figure 22, at room temperature, in the DNA hydrogel system, on the basis of LC/MS method, investigate the accuracy of the test by using nanoparticles, with the output pressure, detecting cocaine in different concentration target.
Figure 23, at room temperature, in the DNA hydrogel system, detecting different concentrations of Adenosine (Adenosine), the reaction time is 5 min.
Figure 24, at room temperature, in the DNA hydrogel system, with Cytidine (Cytidine and Uridine (Uridine) as the negative control, testing the selectivity of the system, the figure shows response to 1 mM Adenosine (Adenosine) and 10 mM Cytidine (Cytidine and Uridine (Uridine), reaction time is 5 min.
Figure 25, at room temperature, in the DNA hydrogel system, detecting for different concentrations of Pb²⁺, the reaction time is 5 min.
Figure 26 at room temperature, in the DNA hydrogel system, Ni²⁺, Mn²⁺, Ca^{2 +}, Co²⁺, Hg²⁺, Fe³⁺, Cu²⁺, Zn²⁺ and Cd²⁺ are used as negative contrast, investigate g the selectivity of the system, the figure shows the response to 1 mM Ni²⁺, Mn^{2 +}, Co²⁺, Co²⁺, Hg²⁺, Fe³⁺, C^{u2+}, Zn²⁺ and Cd²⁺ and 400 nM Pb²⁺, the reaction time is 5 min.
Figure 27, at room temperature, in the DNA hydrogel system, used for testing different concentrations of ochratoxin A (OTA), the reaction time is 5 min.
Figure 28, at room temperature, in the DNA hydrogel system, aflatoxin M1 (AFM1), corn gibberellic ketene (zearalenone, ZEN), T-2 toxin(T-2), aspergillus toxin called patulin (patulin, PAT), citrinin (citrinin, CIT), ochre and aspergillus toxin B (OTB), noise aspergillus toxin (sterigmatocystin on STC are used as negative control, investigate the selectivity of the system, the figure shows the response to 1 mM AFM1, ZEN, T - 2, PAT, CIT, OTB, on STC, and 25 mu M ochratoxin A (OTA), the reaction time is 5 min.
Figure 29, at room temperature, in functional DNA sensing system, investigate the effect of different DNA numbers modified on the nanoparticles to the sensitivity of air pressure meter, by adenosine system, for example, the reaction time is 1.5 hr.
Figure 30 at room temperature, in functional DNA sensor system, detecting for different concentrations of Adenosine (Adenosine), the reaction time is 1.5 hr.

### Preferred embodiments of the invention

### Embodiment 1: the synthesis of acrylic acid and phosphate amide monomer

The synthesis of acrylic acid and phosphate amide monomer is divided into three steps: 1) 6 - amino - 1 - hexanol (1 g, 8.53 mmol), triethylamine (2.36 mL, 17 mmol) added into 100 mL methylene chloride in an ice bath, chase anaerobic conditions in the absence of water and oxygen, drop in methyl acryloyl chloride (2.67 g, 2.55 mmol), the entire system mixing for 2 hours at room temperature. 2) solvent dried by centrifugation under vacuum, add 10 mL ethanol and 15% NaOH (4 mL) into the product, then the product selective hydrolysis into N - (6 - hydroxyhexyl) methacrylamide. The N - (6 - hydroxyhexyl) methacrylamide is separated in silica gel column with ethyl acetate, after separation it begin the next phase of the reaction. 3) after purification, N - (6 - hydroxyhexyl) methacrylamide (0.50 g, 2.70 mmol) is dissolved in 10 mL anhydrous methylene chloride , under the condition of 0 °C, N, N - diisopropyl ethylamine (DIPEA, 0.98 g, 7.5mmol) is dropped in, then 2 - cyanoethylated N, N - diisopropyl chlorinated and phosphoramide (0.87 mL, 3.25 mmol) is dropped in, react at room temperature for 2 hours. After solvent was dried under an centrifuging-vacuum condition, product is eluted on silica gel column with the eluent of ethyl acetate, petroleum ether, triethylamine 40:60:3. The final product is colorless oily liquid. The final product nuclear magnetic characterization data is as follows: ¹H NMR (CDCl₃) : 5.92 (br, 1H), 5.63 (m, 1H), 5.27 (m. 1H), 3.86-3.72 (m, 2H), 3.66-3.49 (m, 4H), 3.30-3.23 (m, 2H), 2.61 (t, 2H), 1.92 (m, 3H), 1.58-1.50 (m, 4H) 1.37-1.32 (m, 4H) 1.17-1.13 (m, 12H). ¹³C NMR (CDCl₃) : 168.6, 140.4, 119.3, 118.0, 63.8, 63.6, 58.6, 58.3, 43.2, 43.1, 39.8, 31.3, 29.7, 26.8, 25.8, 24.9, 24.8, 24.7, 19.0. 31P (CDCl3) : 148.

### Embodiment 2 synthesis and purification of the nucleic acids modified by methyl allyl group, biotin groups and thiol groups

Traditional CPG is used as solid carrier, and single DNA bases is used as raw material, then strand A, strand B, linker-aptamer, detection DNA, ELISA DNA are synthesized from the 3' end to the 5' end on DNA synthesizer, finally the 5' end of the strand of A and B are modified (modification is on the synthesizer) by the acrylic acid and phosphate amide monomer synthesized in embodiment 1, detection DNA is modified by thiol, detailed synthetic sequences are shown in table 1, table 2 and table 3. the CPG modified by Biotin modify is used as solid phase carrier, single DNA bases are used as raw material, capture DNA is synthesized from the 3' end to 5 end on the DNA synthesizer, detailed synthetic sequences are shown in table 2. After the synthesis, transferring the CPG to 2 mL Eppendorf tube of clean and sterilization, add 0.5 mL solution which formed by methylamine: ammonia = 1:1, ammonia solution under 65 °Cfor 30 min, so that the DNA can be cut off the CPG. After Ammonia solution, get supernatant, and use a small amount of ultrapure water cleaning the CPG, combine the supernatant. Add 2.5 times the volume of frozen anhydrous ethanol and 0.1 times the volume of 3 mol/L NaCl to the system, in - 20 °C refrigerator for 30 min alcohol precipitation. After the alcohol precipitation, under 14000 RPM speed centrifuge for 10 min, abandon the supernatant. the coarse product obtained then is dissolved in pH 8, 0.1 mol/L acetate triethylamine (TEAA), using reversed-phase high performance liquid chromatograph for purification. products after reverse phase - HPLC purification then is vacuum dried. Besides the DNA modified by mercapto, the other DNA dissolved in 80% acetic acid to remove DMT, react after 30 min, dry and then dissolve in ultrapure water, desalination processing by using gel filtration column.Use ultraviolet-visible spectrophotometer to determine the absorbance of nucleic acid at 260 nm, calculate the corresponding amount of substance and density according to the extinction coefficient of the DNA. After the quantitative concentration, vacuum concentration. After 5' thiol modified DNA being purified in HPLC, add pH 6.5 0.1 M TEAA to dissolve DNA, detect A260, and diluted to A260 = 100, record total volume as V; then add 0.15 V 1 M AgNO3, blending, react in room temperature response for 30 min; Add 1 M 0.20 V DTT (dithiothreitol), blending, react at room temperature for 5 min. Centrifugal, take supernatant (DTT complex), washing precipitation with 1 v 0.1 M TEAA, and washing liquid is gathered together, Using gel filtration column for desalination processing. Use ultraviolet-visible spectrophotometer to detect the nucleic acid absorbance at 260 nm, calculate the corresponding amount of substance and density according to the extinction coefficient of the DNA. After the quantitation, the solution is concentrated under vacuum.

**Table 1 DNA sequence used in embodiment 2**

| **name** | **sequence** |
|---|---|
| Cocaine Linker-Apt | 5'-ACT CAT CTG TGA ATC TCG GGA GAC AAG GAT AAA TCC TTC AATGAA GTG GGT CTC CC-3' (underlined part is the sequence of aptamer) |
| Cocaine Strand A | 5'-acrydite-AAA ATC ACA GAT GAG T-3' |
| Cocaine Strand B | 5'-acrydite-AAA AGT CTC CCG AGA T-3' |
| Adenosine Linker-Apt | 5'-ACT CAT CTG TGAAGA GAA CCT GGG GGA GTA TTG CGG AGG AAG GT-3' (underlined part is the sequence of aptamer) |
| Adenosine Strand A | 5'-acrydite-AAA ATC ACA GAT GAG T-3' |
| Adenosine Strand B | 5'-acrydite-AAA ACC CAG GTT CTC T-3' |
| Pb²⁺ Substrate strand | 5'-CTA CAA AAA CAC ATA CTC ACT ATr AGG AAG AGA TGA TCC ACA TTT TCA CAG -3' |
| Pb²⁺ Enzyme strand | 5'-CAT CTC TGA AGT AGC GCC GCC GTA TAG T -3' |
| Pb²⁺ Strand A | 5'-acrydite- CTG TGA AAA TGT GG-3' |
| Pb²⁺ Strand B | 5'-acrydite- ATG TGT TTT TGT AG -3' |
| OTA Linker | 5'-CAG TGT CTG TGA GTA TGC GAT CGG GTG TGG GTG GCG TAA AGG GAG CAT CGG ACA-3'(underlined part is the sequence of aptamer) |
| OTA Strand A | 5'-acrydite-CAC AGA CAC TG -3' |
| OTA Strand B | 5'-acrydite-CCG ATC GCA TA -3' |

**Table 2 DNA sequence used in embodiment 2**

| **name** | **sequence** |
|---|---|
| Biotin-DNA | 5 -TCA CAG ATG AGT AAAAAA-AAAAAA-Biotin-3 |
| Cocaine-Aptamer | 5 -TTT TTT ACT CAT CTG TGA ATC TCG GGA GAC AAG GAT AAA TCC TTC AAT GAA GTG GGT CTC CC-3 (underlined part is the sequence of aptamer) |
| Cocaine-DNA-SH | 5 -HS-AAA AAA AAA AAA GTC TCC CGA GAT-3 |
| Adenosine-Aptamer | 5 -TTT TTT ACT CAT CTG TGA AGA GAA CCT GGG GGA GTA TTG CGG AGG AAG GT-3 |
| | (underlined part is the sequence of aptamer) |
| Adenosine-DNA- SH | 5 -HS-AAA AAA AAA AAA CCC AGG TTC TCT-3 |

**Table 3 DNA sequence used in embodiment 2**

| **name** | **sequence** |
|---|---|
| ELISA DNA | 5'-TTTTTTTTTT- PEG - PEG - PEG - Biotin-3' |

### Embodiment 3: double-antibody sandwich preparation

Preparation of the H5N1 avian flu virus HA protein, for example, capture antibody is packaged in 96-well plate, the concentration is 2.0 µg/mL, the volume is 100 µL, seal the plate, 4 °C overnight. Washing three times using Wash Buffer. Add 300 µL blocking liquid for at least 1 hr at room temperature for sealing. Add 100 µL antigens to be detected with different concentrations, incubation 2 hr at room temperature, after washing three times, add 100 µL, 1. 5 µg/mL detection antibody, incubation at room temperature for 1 hr, form double-antibody sandwich structure.

### Embodiment 4: feasibility verifying test of conventional ELISA protein system

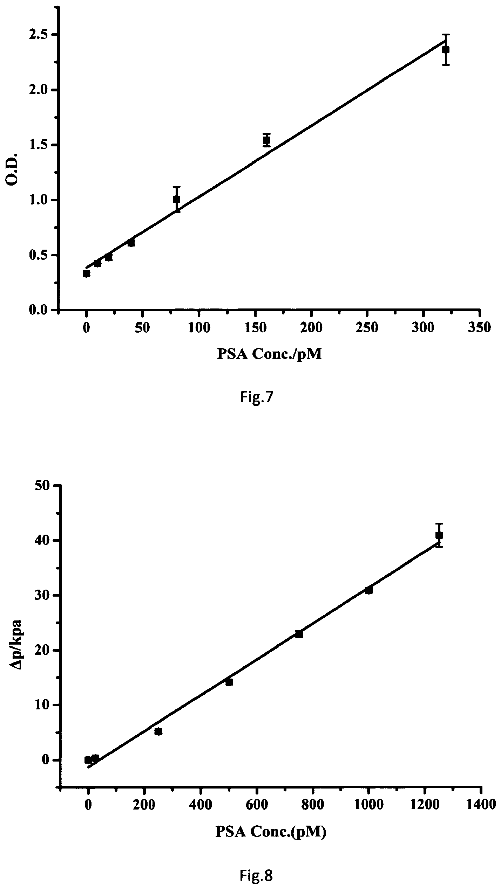

Conventional ELISA reaction process is that, firstly the capture antibody is coated, protein and polystyrene solid phase carrier are combined by hydrophobic force. Then respectively add antigen, biotinylated detection antibody, SA - HRP complexes and the substrate, under the reaction of the substrate, by determining absorption of detecting targets, the result shown in figure 7, figure 10 and figure 15.

### Embodiment 5: preparation of biotinylated hydrogen peroxide enzyme (Catalase)

buffer used in biotinylating is: 0.1 M sodium bicarbonate, pH 8.4. 1 mg/ml BNHS (biotin - N - Amber imine esters) is slowly dropped into 10 mg/ml hydrogen peroxide enzyme (Catalase), control the final reaction volume ratio of 8:1. , shaking and mixed, react at room temperature for 4 h. Ultrafiltration centrifugal, remove free BNHS, biotinylated hydrogen peroxide enzyme (Catalase) thus obtained.

### Embodiment 6: ELISA system test- signal amplification molecules is hydrogen peroxide enzyme (Catalase)

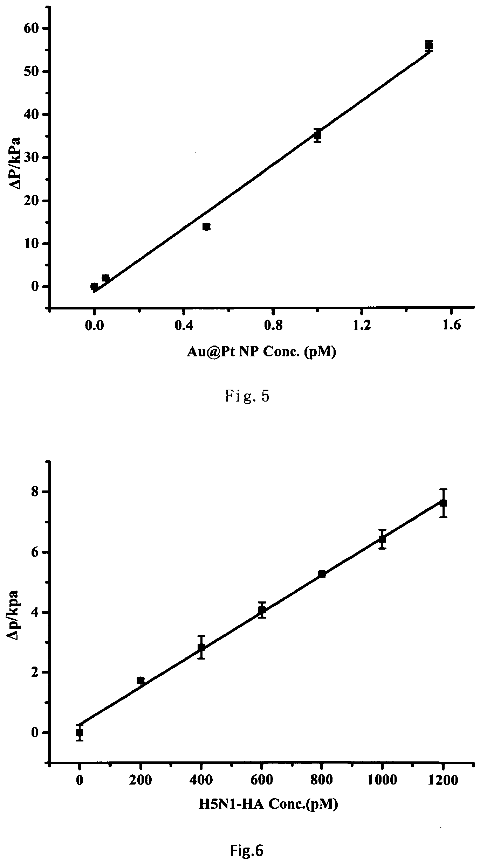

Add 100 µL, 1 µg/mL Streptavidin into the microcavity of 96-well palate having prepared double-antibody sandwich structure, incubation at room temperature for 1 hr, make it combined with the biotin on the detection antibody; Add 100 µL, 20 nm Catalase - biotin, room temperature incubation for 1 hr, wash away excess hydrogen peroxide enzyme; add solution phase in the substrates into 96 - well plate, cover the reaction cover to seal.the Signal amplification molecular, hydrogen peroxide enzyme act to the substrates in solution phase, produce signal molecule which can be read by the air pressure meter. According to pressure meter readings, record the test results. The results are shown in figure 6.

Figure 6 is at room temperature, ELISA system, use hydrogen peroxide enzyme to detect target protein, the H5N1 avian flu virus HA protein with different concentrations. capture antibody is coated in 96-well plates, its concentration is 2 µg/mL, its volume is 100 µL, then seal the plate, 4°C overnight. Then wash three times using Wash Buffer. Add 300 µL blocking liquid and block at room temperature for at least 1 hr. Add the 100 µL antigens being detected with different concentrations, incubated at room temperature for 2 hr, after washing three times, add 100 µL, 1.5 µg/mL detection antibody, incubated at room temperature for 1 hr, form double-antibody sandwich structure, then wash out the detection antibody; add 100 µL, 1 µg/mL Streptavidin, and incubate at room temperature for 1 hr, make it being combinate to the biotin on the detection antibody; Add 100 µL, 20 nM Catalase - biotin, incubate at room temperature for 1 hr, wash away excess hydrogen peroxide enzyme; add solution phase, the substrates H202 (0. 31 M) into 96 - well plates, cover the reaction cover, make it being sealed. Signal amplification molecular, the hydrogen peroxide enzyme act in the solution phase substrates, produce signal molecules which can be read by the air pressure meter. Control the reaction time for 1 hr. Record test results according to the readings of the air pressure meter.

### Embodiment 7: ELISA system test- signal amplification molecule is Au @ PtNPs

Add 100 µL, 1 µg/mL Streptavidin into the microcavity of 96-well palate having prepared double-antibody sandwich structure, incubation at room temperature for 1 hr, make it combined with the biotin on the detection antibody; Add 100 µL, 2. 5nM Au@PtNPs-DNA-biotin, room temperature incubation for 1 hr, wash away excess nanoparticles; add solution phase in the substrates into 96 - well plate, cover the reaction cover to seal.the Signal amplification molecular, Au@PtNPs act to the substrates in solution phase, produce signal molecule which can be read by the air pressure meter. According to pressure meter readings, record the test results. The results are shown in figure 8, figure 9, figure 11, figure 12 and figure 13, figure 14, figure 16, figure 17.

Figure 8 is at room temperature, ELISA system, use nanoparticles to detect target protein, PSA recombinant proteins with different concentrations. capture antibody package is coated in 96 - well plate, the concentration is 2.0 µg/mL, the volume is 100 µL, seal the plate, 4°C overnight. Wash three times by Wash Buffer. add 300 µL blocking liquid to block at least 1 hr at room temperature. Add 100 µL of antigens to be detected with different concentrations, incubate 2 hr at room temperature, after washing three times, add 100 µL, 0.2 µg/mL detection antibody, incubate 1 hr at room temperature, form the double-antibody sandwich structure, wash away the detection antibody; add 100 µL, 1 µg/mL Streptavidin, incubate at room temperature for 1 hr, make it the combined with the biotin on the detection antibody; add 100 µL, 0.625 nM Au @ PtNPs - DNA - biotin, incubate 1 hr at room temperature, wash away excess nanoparticles; add substrates in solution phase into the 96 - well plates, cover the reaction cover, make it being sealed. Molecular signal amplification Au @ PtNPs act to the substrates in solution phase, produce signal molecule which can be read by the air pressure meter. Control the reaction time for 1 hr. Record test results according to the readings of the air pressure meter.

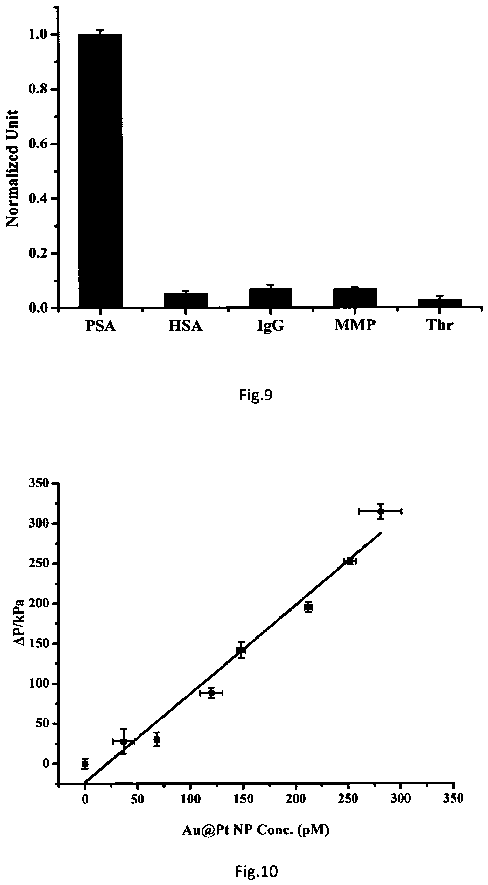

Figure 9 is at room temperature, ELISA system, HSA, IgG, MMP, Thr are used as negative control, investigate the PSA selective of the system, the figure shows the response for 120 µm HSA, IgG, MMP, Thr, PSA, the reaction time is 1 hr.

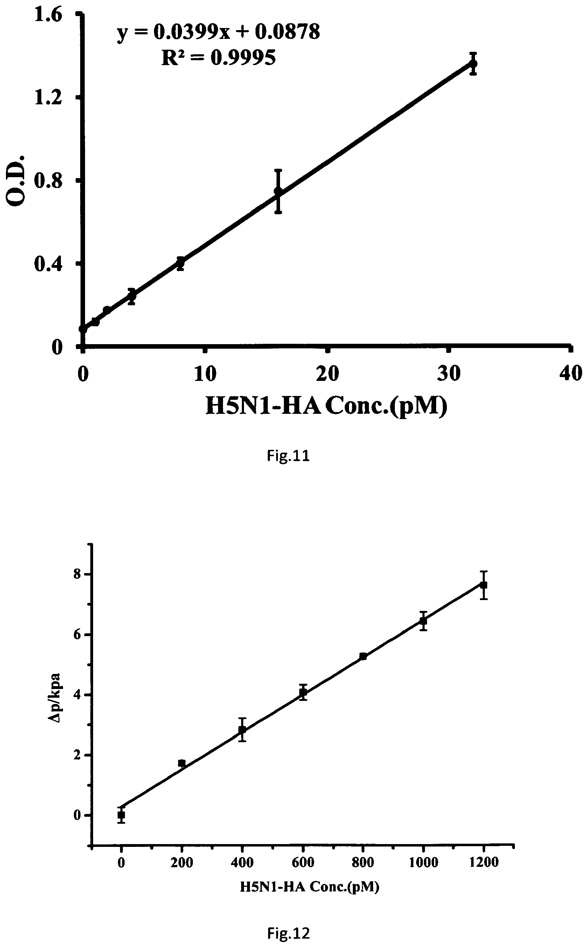

Figure 11 is at room temperature, ELISA system, use nanoparticles to detect target protein, H5N1 avian flu virus HA protein with different concentrations. capture antibody package is coated in 96 - well plate, the concentration is 2.0 µg/mL, the volume is 100 µL, seal the plate, 4°C overnight. Wash three times by Wash Buffer. add 300 µL blocking liquid to block at least 1 hr at room temperature. Add 100 µL of antigens to be detected with different concentrations, incubate 2 hr at room temperature, after washing three times, add 100 µL, 0.2 µg/mL detection antibody, incubate 1 hr at room temperature, form the double-antibody sandwich structure, wash away the detection antibody; add 100 µL, 1 µg/mL Streptavidin, incubate at room temperature for 1 hr, make it the combined with the biotin on the detection antibody; add 100 µL, 2.5nMAu@PtAuNPs-DNA-biotin, incubate 1 hr at room temperature, wash away excess nanoparticles; add substrates in solution phase into the 96 - well plates, cover the reaction cover, make it being sealed. Molecular signal amplification Au@PtAuNPs act to the substrates in solution phase, produce signal molecule which can be read by the air pressure meter. Control the reaction time for 1 hr. Record test results according to the readings of the air pressure meter.

Figure 12 is at room temperature, ELISA system, IgG, HSA, H3N2, SARS are used as negative control, investigate the selectivity of the system to the H5N1 avian flu virus HA protein, the figure shows responses to 120 µm HSA, H3N2, H5N1, IgG SARS, the reaction time is 1 hr.

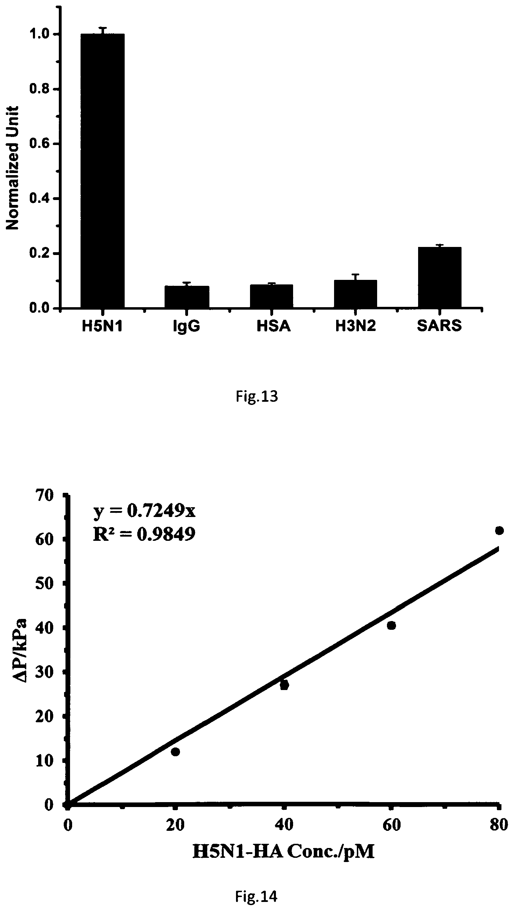

Figure 13 is at room temperature, ELISA system, in the context of serum HA, by adding different concentrations of H5N1 bird flu virus, with nanoparticles as signal amplification molecules, detect target protein, the H5N1 avian flu virus HA protein with different concentration.

Figure 14 is at room temperature, ELISA system, detect target protein, the H5N1 avian flu virus HA protein with different concentrations. the protein samples containing different concentrations of H5N1 avian flu virus HA were divided into two groups, one group is detected by conventional ELISA in a standard way, another group use nanoparticles as signal amplification method to detect target protein, the H5N1 avian flu virus HA protein with different concentrations, investigate the accuracy of the method of the invention.

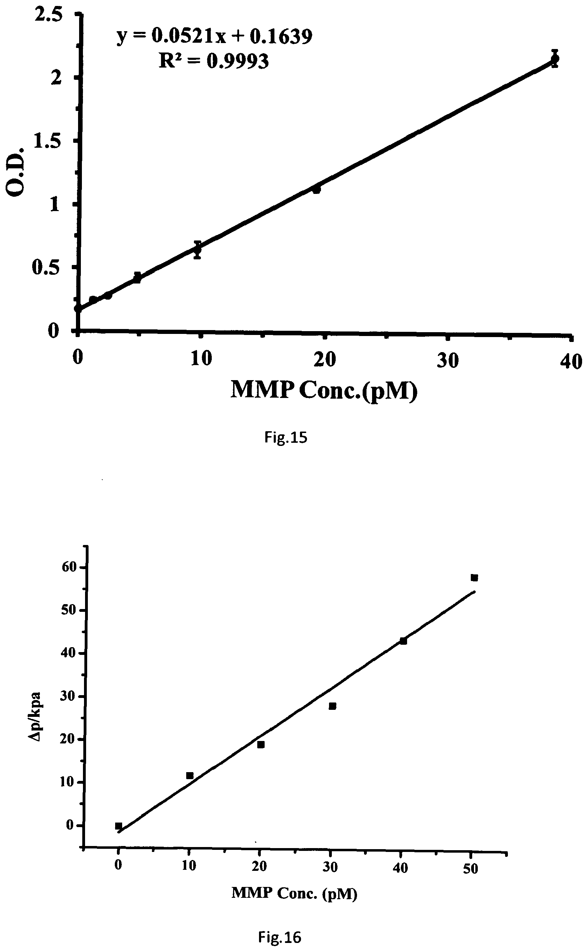

Figure 16 is at room temperature, ELISA system, use nanoparticles to detect target protein, reorganization of human MMP9 protein with different concentrations. capture antibody package is coated in 96 - well plate, the concentration is 2.0 µg/mL, the volume is 100 µL, seal the plate, 4°C overnight. Wash three times by Wash Buffer. add 300 µL blocking liquid (2%BSA), block at least 1 hr at room temperature. Add 100 µL of antigens to be detected with different concentrations, incubate 2 hr at room temperature, after washing three times, add 100 µL, 0.25 µg/mL detection antibody, incubate 1 hr at room temperature, form the double-antibody sandwich structure, wash away the detection antibody; (4) add 100 µL, 1 µg/mL Streptavidin, incubate at room temperature for 1 hr, make it the combined with the biotin on the detection antibody; (5)add 100 µL, 0.625 nM Au @ PtNPs- DNA-biotin, incubate 1 hr at room temperature, wash away excess nanoparticles; (6)add substrates in solution phase into the 96 - well plates, cover the reaction cover, make it being sealed. Molecular signal amplification Au @ PtNPs act to the substrates in solution phase, produce signal molecule which can be read by the air pressure meter. Control the reaction time for 1 hr. Record test results according to the readings of the air pressure meter.

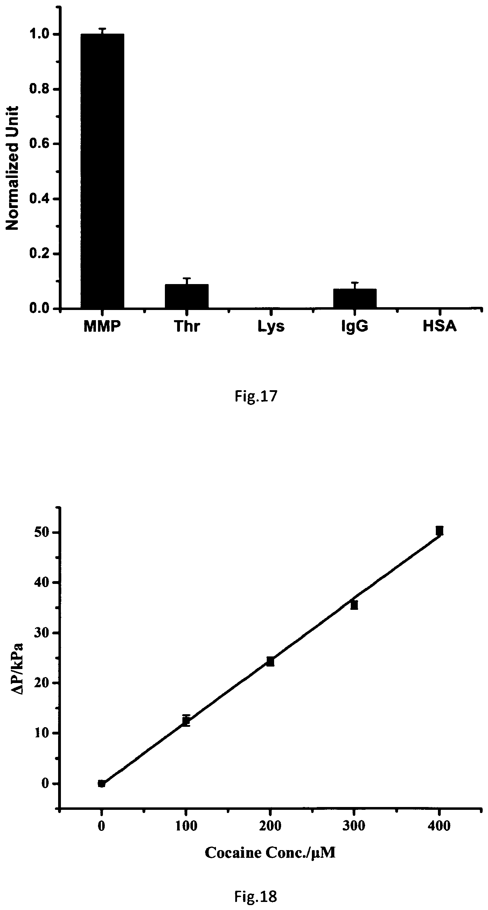

Figure 17 is at room temperature, ELISA system, Thr, Lys, IgG, HSA are used as negative control, investigate the MMP selective in the system, the figure shows the response for 120 µMMMP, Thr, Lys, IgG, HSA, the reaction time is 1 hr.

### Embodiment 8: the preparation of hydrogels

Respectively dissolve the strand A and strand B into ultrapure water to form stock solution of high concentration DNA. Respectively prepare 10% ammonium persulfate and 5% TEMED (N, N, N', N'- tetramethyl ethylenediamine), i.e. 0.05 g ammonium persulfate dissolved to 0.5 mL ultrapure water and 25 µl TEMED dissolved in 0.5 mL ultrapure water. DNA and the acrylamide with the final concentration of 4% are mixed as mixture, vacuum degassing in the vacuum dryer for 10 min. add fresh compound initiator (ammonium persulfate) and accelerator (TEMED) with the final concentration of 1.4%, put the reaction system in the vacuum dryer after blending, under the condition of 30°C, vacuum reaction 15 min, get linear polymer product PS-A, PS -B of the strand A and strand B. Mix PS-A and PS- B according to 1:1, add 1 µL, 10 x reaction buffer (PB buffer for cocaine: 77 mM Na₂HPO₄, 23 mM NaH₂PO₄, 50 mM NaCl, 5 mM MgCl₂, pH 7.3; Tris - HCl buffer for adenosine: 10 mM Tris-HCl, 100 mM NaCl, 10 mM MgCl₂, pH 8.0; using Tris-CH3COOH buffer for Pb²⁺: 10 mM Tris-HCl, 300 mM NaCl, pH 8.0; Tris - HCl buffer for ochratoxin A: 10 mM Tris-HCl, 120 mM NaCl, 25 mM KCl, 20 mM CaCl₂, pH 8.5), then add 2 µl, 23 nM Au @ PtNPs blend by oscillation, then add PS-A,PS- B and linker aptamer in the ratio of is 1:1:0. 6. Repeat oscillation and the heating process, until get the three-dimensional crosslinked hydrogel coated with Au @ PtNPs. The forward reaction is process in dry bath, keep step heating at 65 °C for 5 min, then cool to room temperature by nature.

### Example 9: steps of DNA hydrogel visual quantitative testing

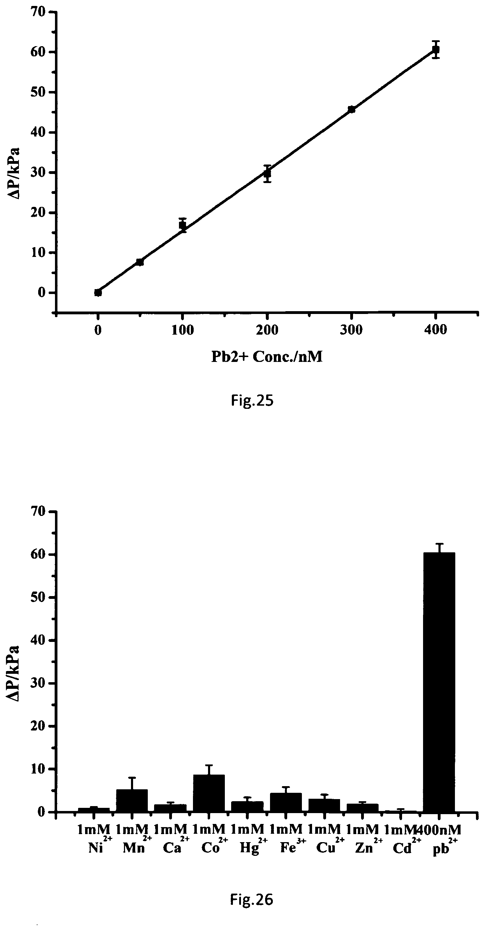

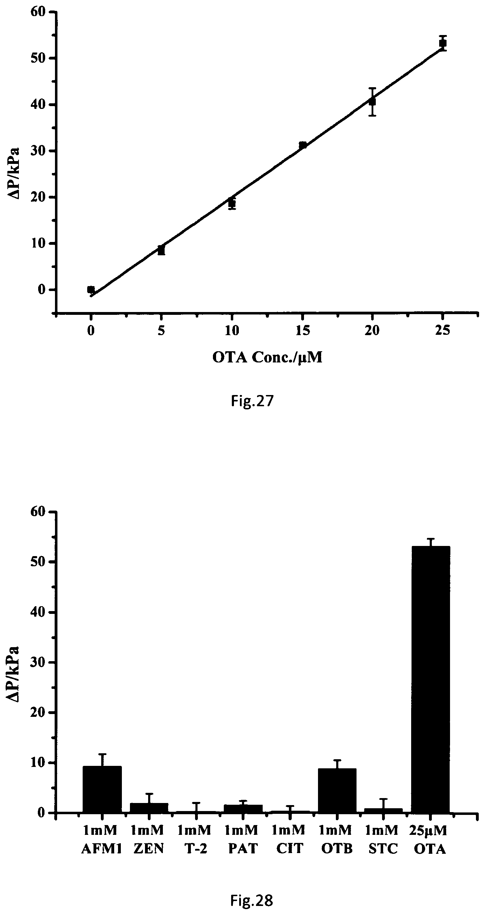

Before using, wash the prepared hydrogel by corresponding 1 x Buffer 3 times, to remove signal amplification molecules residue in centrifugal tube wall and on the surface of the hydrogel, and then remove the water. Prepare targets detection by use of buffer solution, and add it to the centrifuge tube containing hydrogel. Transfer the centrifuge tube to shaker, at 30 °C, fully reactions under the speed of 150 RPM. Collected the supernatant after the reaction, in 200 µ L homemade reaction tube, add 50 µL supernatant respectively with 50 µ LH202, control of reaction time, read and record the data according to the changes of gas pressure, the results are shown in figure 18, figure 19 and figure 20, figure 21, figure 22, figure 23, 24, 25" FIG. 26 FIG. 27 and FIG. 28.

Figure 18, at room temperature, in the DNA hydrogel system, for the detection of different concentrations of Cocaine. DNA hydrogel peptization experiment is in PB buffer (77 mM Na₂HPO₄ 23 mM NaH₂PO₄, 50 mM NaCl, 5 mM MgCl₂, pH 7. 3) in 30°C, 150 RPM to react 1 hr. Collect the supernatant, in the 200 mu L homemade reaction tube, respectively add 50 µL supernatant and 50 µLH202, after reaction 5 min, measuring the pressure, and record the data. The pressure difference value of measured value to value of blank group (0 µM group), was calculated for plotting.

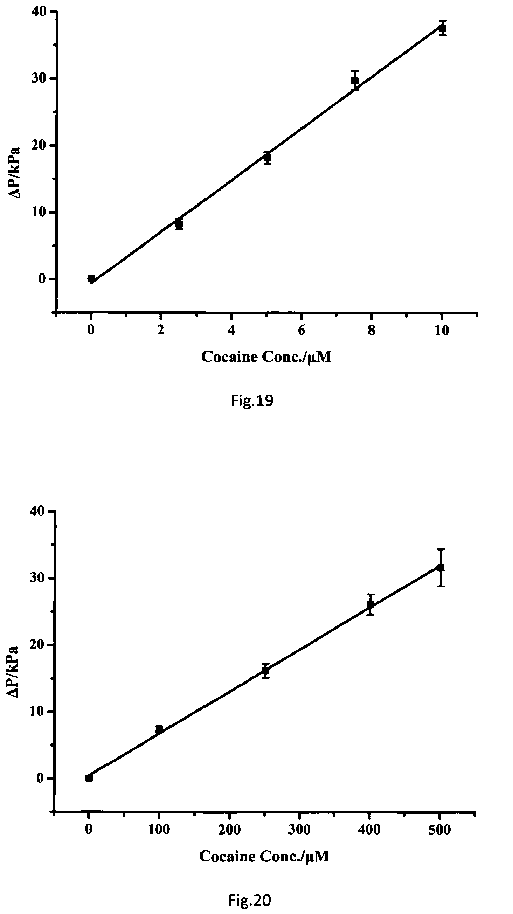

Figure 19, at room temperature, in the DNA hydrogel system, for the detection of different concentrations of Cocaine. DNA hydrogel peptization experiment is in PB buffer (77 mM Na₂HPO₄, 23 mM NaH₂PO₄, 50 mM NaCl, 5 mM MgCl₂, pH 7.3) in 30°C, 150 RPM to react 1 hr. Collect the supernatant, in the 200 mu L homemade reaction tube, respectively add 50 µL supernatant and 50 µLH2O2, after reaction 15 min, measuring the pressure, and record the data. With 0 µM difference between the experimental group, drawing. Through the extension of the reaction time, to realize lower limit of detection for the system.

Figure 20, at room temperature, in the DNA hydrogel system, in the actual samples in the urine, for the detection of different concentrations of Cocaine. DNA hydrogel peptization experiment is in PB buffer (77 mM Na₂HPO₄, 23 mM NaH₂PO₄, 50 mM NaCl, 5 mM MgCl₂, pH 7.3) in 30°C, 150 RPM to react 1 hr. Collect the supernatant, in the 200 µL homemade reaction tube, respectively add 50 µL supernatant and 50 µLH202, after reaction 5 min, measuring the pressure, and record the data. The difference value of measured pressure value to 0 µM experimental group was calculated, and plotted to sample concentration

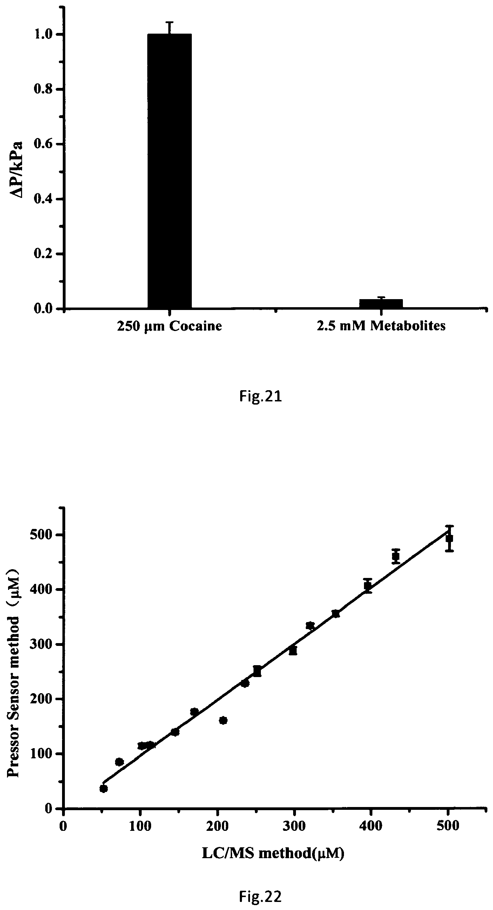

Figure 21, at room temperature, in the DNA hydrogel system, benzoyl ecgonine and methyl ecgonine of cocaine metabolites are used as negative control, investigate the selectivity of the system, the figure shows the response to 250 µm cocaine and 2.5 mM benzoyl ecgonine and methyl ecgonine, DNA hydrogel dispergation experiment is proceed in PB buffer (77 mM Na₂HPO₄, 23 mM NaH₂PO₄, 50 mM NaCl, 5 mM MgCl₂, pH 7. 3) in 30 °C, 150 RPM reaction 1 hr. Collect supernatant, in the 200 µL homemade reaction tube, respectively add 50 µL reaction solution with 50 µL H202, react for 5 min.

Figure 22, at room temperature, in the DNA hydrogel system, in the actual samples of urine, detecting for different concentrations of Cocaine. Samples are divided into two copies, one copy of LC/MS detection in a standard way, another to mentioned method of the invention, output by air pressure, detect the target cocaine of different concentrations, investigate the accuracy of the method.

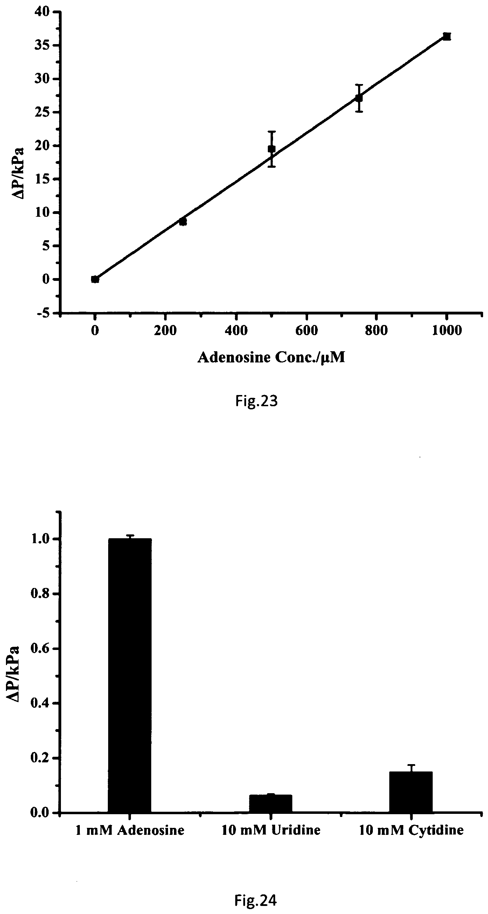

Figure 23, at room temperature, in the DNA hydrogel system, detecting for Adenosine with different concentrations, the reaction time is 5 min. Changing the DNA sequence of hydrogel according to the target, can realize the detecting for corresponding target. DNA hydrogel peptization experiment is in Tris-HCl buffer (10 mMTris-HCl, 100 mM NaCl, 10 mM MgCl₂, pH 8.0) in 30 °C, 150 RPM react for 2 hr. Collect the supernatant, in the 200 mu L homemade reaction tube, respectively add 50 µL supernatant and 50 µL H202, after reacting for 5 min, measure the pressure, and record the data. The difference value of measured pressure value to 0 µM experimental group was calculated, and plotted to sample concentration.

Figure 24, at room temperature, in the DNA hydrogel system, Cytidine and Uridine are used as the negative control, investigate the selectivity of the system, the figure shows response to 1 mM Adenosine and 10 mM Cytidine, Uridine, response time is 5 min. DNA hydrogel peptization experiment is in Tris-HCl buffer (10 mMTris - HCl, 100 mM NaCl, 10 mM MgCl2, pH 8.0) in 30°C, 150 RPM react for 2 hr. Collect the supernatant, in the 200 µL homemade reaction tube, respectively add 50 µL supernatant and 50 µL H2O2, react 5 min, then measure the pressure, and record the data. The difference value of measured pressure value to 0 µM experimental group was calculated, and plotted to sample concentration.

Figure 25, at room temperature, in the DNA hydrogel system, for detecting of Pb2 + with different concentrations, the reaction time is 5 min. DNA hydrogel peptization experiment is in Tris-HCl buffer (10 mMTris - CH3COOH, Tris HCl - 10 mM, 300 mM NaCl, pH 8.0) in 30°C, 150 RPM 2.5 hr. Collect supernatant, in the 200 µL homemade reaction tube, respectively add 50 µL supernatant and 50 µL H202, react for 5 min, then measure the pressure, and record the data. The difference value of measured pressure value to 0 µM experimental group was calculated, and plotted to sample concentration.

Figure 26, at room temperature, in the DNA the hydrogel system, Ni²⁺, M²⁺, C²⁺, Co²⁺, Hg²⁺, Fe³⁺, Cu²⁺, Zn²⁺ and Cd²⁺ are used as negative control, investigate the selectivity of the system, the figure shows the response to 1 mM Ni²⁺, Mn²⁺, Ca²⁺ Co²⁺, Hg²⁺, Fe³⁺, Cu²⁺, Zn²⁺, Cd²⁺ and 400 nM Pb²⁺, the reaction time is 5 min. DNA hydrogel peptization experiment is in Tris-HCl buffer (10 mMTris-CH₃COOH, 10 mM Tris-HCl, 300 mM NaCl, pH 8.0) in 30°C, 150 RPM, 2.5 hr. Collect supernatant, in the 200 µL homemade reaction in vitro, respectively add 50 µL supernatant and 50 µL H202, react for 5 min, measure the pressure, and record the data. The difference value of measured pressure value to 0 µM experimental group was calculated, and plotted to sample concentration.

Figure 27, at room temperature, in the DNA hydrogel system, being used for detecting ochratoxin A (OTA) with different concentrations, the reacting time is 5 min. DNA hydrogel peptization experiment is in Tris-HCl buffer (10 mMTris- HCl, 120 mM NaCl, 25 mM KCl, 20 mM CaCl₂, pH 8.5) in 30 °C, 150 RPM 1.5 hr. Collect supernatant, in the 200 µL homemade reaction tube, respectively add 50 µL supernatant and 50 µL H202, react for 5 min, measure the pressure, and record the data. The difference value of measured pressure value to 0 µM experimental group was calculated, and plotted to sample concentration.

Figure 28, at room temperature, in the DNA hydrogel system, aflatoxin M1 (AFM1), corn gibberellic ketene (zearalenone, ZEN), T-2 toxin(T-2), aspergillus toxin called patulin (patulin, PAT), citrinin (citrinin, CIT), ochre and aspergillus toxin B (OTB), noise aspergillus toxin (sterigmatocystin on STC) are used as negative control, investigate the selectivity of the system, the figure shows the response to **1 mM AFM1, 2EA, T-2, PAT, CIT, OTB, SOER** and 25 µ m ochratoxin A (OTA), the reaction time is 5 min. DNA hydrogel peptization experiment is in Tris-HCl buffer (**10 mMTris- HCl, 120 mM NaCl, 25 mM KCl, 20 mM CaCl2, pH 8.5**) in 30 °C, 150 RPM 1. 5 hr. Collect supernatant, in the 200 µL homemade reaction tube, respectively add 50 µL supernatant and 50 µL H202, react for 5 min, measure the pressure, and record the data. The difference value of measured pressure value to 0 µM experimental group was calculated, and plotted to sample concentration.

### Embodiment 10: preparation of detection DNA

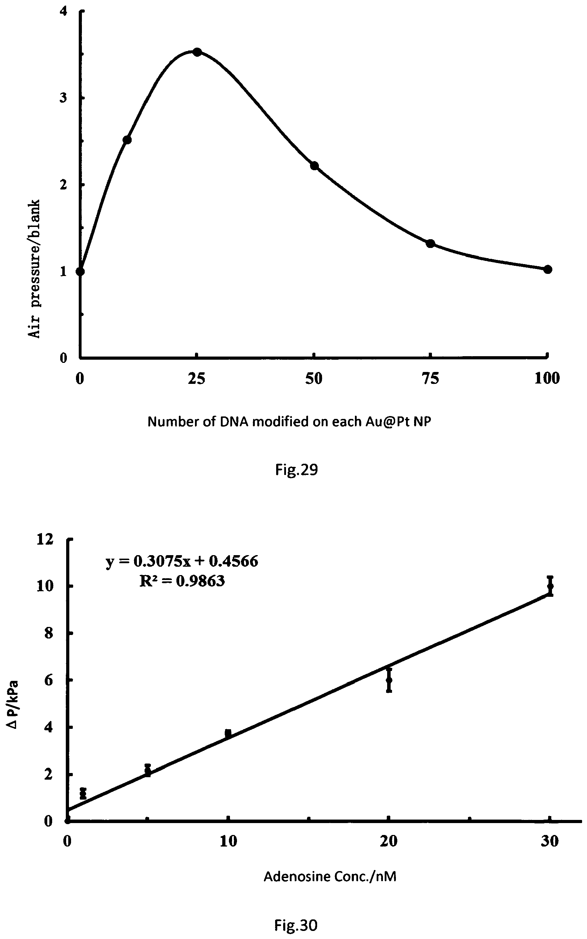

Take 1 ml solution of 13 nm Au @ Pt - Au, 14000 r/m centrifugal, remove the supernatant, add 1 ml Buffer, resuspended, repeated three times. Add 100 µ L F127 whose volume concentration is 10%, blending. Add 8 µL DNA-SH, blending. Add 50 µL 0.2 M H3P04, blending. 37 °C, rotation reaction for 1 h., 14000 r/m centrifugal, remove the supernatant, add 1 ml Buffer resuspend, repeated three times. By control the DNA - SH concentration, Au @ PtNPs modified with different number of DNA can be prepared, the results are shown in figure 29.

Figure 29, under the condition of 25 °C, in functional DNA sensing system, investigate the different DNA number modified on nanoparticles effect to the sensitivity of air pressure meter, adenosine system, for example, the reaction time is 1.5 hr. Take 150 µl Streptavidin to modify magnetic beads, use 1 mL of PBS-T Buffer 1 time to clean; Heavy suspension in 950 µl PBS Buffer-T, add 25 µL 4µM Biotin - DNA and 25 µL 4 µM Ade-Apt, 200 RPM, 25 °C shake for 30 min; wash by PBS Buffer-T three times, wach time 1 mL; Take 600µl suspending liquid containing magnetic beads modified by Apt, add 200µl **25 nM Pt-DNA** modified with different number of DNA and 300µl PBS-T Buffer (increasing the reaction volume, is advantageous to the blending of magnetic beads), 25 °C blending for 30 min; use PBS-T Buffer to wash 6 times, each time 1 mL; suspending in 300µL PBS_t Buffer, respectively divide into two centrifugal tubes, each centrifugal tube including 50 µL, abandon supernatant, each group is added in adenosine with the final concentration 100 nm, the control group is added in PBS-T Buffer, final volume are 50 µL, 25 °C blending for 30 min; put 10 µL supernatant + 40 µL PBS buffer + 50 µL H202 in air pressure meter reaction tube, blending with a suction gun 10 times, then covered tightly . React for 1. 5h, then detect the pressure inside the system. Each sample has four parallel experiments. **The signal/blank ratio was obtained by the dividing the pressure value of experiment group by the pressure value of blank group.**

### Embodiment 11: the preparation of functional DNA sandwich structure

Take 15 µL magnetic beads modified by Streptavidin (strand mildew avidin), wash with 100 µL PBS -t Buffer 1 time; heavy Suspended in 98 µL PBS-T Buffer, add 2 µL 4 µM Biotin-DNA, 200 RPM, 25 °C shake for 30 min; wash by PBS-T Buffer, each time 100 µL ; Heavy suspension in 80 µL PBS- T Buffer, add 20 µL 330 nM Aptamer, 200 RPM, 25 °C shake for 30 min; Each time, wash with PBS-T Buffer three times each time 100 µL; add 240 µL 2. 5 nM DNA-Pt, 200 RPM, 25 °C shake for 30 min, then get the functional DNA sandwich structure.

### Embodiment 12: visual quantitative detection of functional DNA

The prepared functional DNA sandwich structure is heavy suspended in 100 µL PBS-T Buffer, add target solution with different concentrations, put on shaking bed, 200 RPM, 25 °C shake for 30 min. After the reaction, take the supernatant into reaction tubes, let Au @ PtNPs catalyzed with H202; In the closing system, the generated 02 is transferred into pressure, using air pressure meter to read, and record the data. The results are shown in figure 30.

Figure 30, under the condition of 25 °C, in the functional DNA sensor system, used for detecting Adenosine with different concentrations, the reaction time is 1.5 hr. Take 150 µL magnetic beads modified by Streptavidin, wash by 1 mL PBS - T Buffer 1 time; Heavy suspension in 950 µL PBS-T Buffer, add 25 µL 4 µM Biotin-DNA and 25 µL Ade - Apt, 200 RPM, 25 °C shake for 30 min; washed by PBS-T Buffer three times, Each time 1 mL; Take 600 µL suspending liquid containing magnetic beads modified by Apt, add 200 µL 25 nM Pt-DNA and 300 µL PBS-T Buffer (increasing the reaction volume, is advantageous to the blending of magnetic beads), 25 °C blending for 30 min; washed by PBS-T Buffer 6 times, each time 1 mL; Heavy suspension in 300 µL PBS-T Buffer, divided into six centrifugal tubes, each centrifugal tube including 50 µL, abandon supernatant, respectively, add **50** µL, **49.5** µL, **47.5** µL, **49.5** µL, **49** µL, **48.5** µL PBS-T buffer into a, b, c, d, e, f, then respectively, add **0** µL, **0.5** µL, **2.5** µL 0.1 µM Adenosine into a, b, c, while add 0.5 µL , 1 µL, 1.5 µL Adenosine into d, e, f, makes the final volume of 50 µL, blending in 25 °C for 30 min; Put 10 µL supernatant + 40 µL LPBS-T Buffer + 50 µL H202 into air pressure meter reaction tube, blending 10 times by a suction gun, and air-sealed tightly. detect the pressure inside the system after 1.5 h. Each sample has four parallel experiments.

The foregoing description of the exemplary embodiment of the invention has been presented for the purposes of illustration and description. It is not intended to be exhaustive or to limit the invention to the precise form disclosed. Many modifications and variations are possible in light of the above teaching. It is intended that the scope of the invention be limited not with detailed description, but rather by the claims appended hereto.

### Industrial applicability

The present invention is to generate large amount of gas molecules by using enzymes or nanometer particles, etc. ; converts target molecules detection signal into gas pressure signal; achieves signal amplification; and finally converts the pressure change into an electrical signal to conduct a reading through a barometer, thereby achieving high-sensitivity quantitative detection.

## Claims

1. A quantitative analysis method based on air pressure measuring, wherein by introducing signal amplification molecules or particles into an air-sealed system by molecular recognition, a large amount of gas molecules are generated by the reaction of the molecular signal amplification molecules or particles catalyzing the substrate, cause the air-sealed system pressure increasing, measure the pressure changes of the reacting detection system to detect the target concentration.

2. The quantitative analysis method based on air pressure measuring according to claim 1, wherein said molecular recognition comprising: identifying or labeling said targets by using molecules with specifically recognizing function, and then the signal molecules or particles are introduced into the detection system through the recognition molecules.

3. The quantitative analysis method based on air pressure measuring according to claim 2, wherein in the target recognizing or labeling by molecular recognition method, said target comprising proteins, nucleic acids, peptides, sugars, lipids, small organic molecules, inorganic ions, cells, bacteria and viruses.

4. The quantitative analysis method based on air pressure measuring according to claim 1, wherein said signal amplification molecules comprising catalase or enzyme which can catalyze substrate to generate gas molecules, the catalytic substrate is the material which can generate gas molecules after being catalyzed, gas molecules are the product after the substrate being catalyzed.

5. The quantitative analysis method based on air pressure measuring according to claim 4, wherein said signal amplification molecules comprising catalase, gold nanoparticles, platinum nanoparticles, gold-platinum nanoparticles or manganese oxide nanoparticles.

6. The quantitative analysis method based on air pressure measuring according to claim 1, wherein in said air-sealed system, said reacting detection system comprising enzyme-linked immunosorbent assay system, DNA hydrogel system or functional DNA sensing system.

7. The quantitative analysis method based on air pressure measuring according to claim 6, wherein in said enzyme-linked immunosorbent assay system, quantitative analysis method based on air pressure measuring comprises the following steps: (1) selecting corresponding capture antibodies and detection antibodies according to the testing antigens; (2) modifying the signal amplification molecules or particles, labeling them with detection antibody molecules, or to be able to specifically conjugate to detection antibodies; (3) coating the solid surface which can be used for enzyme-linked immunosorbent assay such as multi-wells plate or magnetic beads or microspheres, adding capture antibodies to let they being conjugated or absorbed onto the solid surface after the coating, and then blocking the surface with blocking solution, adding the antigen solution to be detected, and add detection antibody to form double-antibody sandwich structure, wash away excess detection antibodies; (4) introducing signal amplification molecules; (5)adding substrate in the multi-wells plate, sealing the wells, signal amplification molecules or particles catalyze the substrate molecules, then a large amount of gas molecules are generated, and producing the pressure variation signal which can be read by air pressure meter; (6) the target molecule concentration is quantitatively measured according to the pressure value changes of the air pressure meter.

8. The quantitative analysis method based on air pressure measuring according to claim 6, wherein in said DNA hydrogel system, quantitative analysis method based on air pressure measuring comprising the following steps: (1) mix two poly-acrylamide-DNA strands, an aptamer and signal amplification molecules to prepare DNA aptamer crosslinked hydrogel containing signal amplification molecules; (2) add solution containing different known concentrations of analyte to the hydrogel, reacting and releasing signal amplification molecules; (3) after the reaction, the supernatant is put into a reaction tube, and make the signal amplification molecules and substrate process catalytic reaction; in an air-sealed system, the large amount of gas make the pressure rise inside the system, use air pressure meter to read, and record the data, then standard curve can be established, thus the content of target in the unknown samples can be detected.

9. The quantitative analysis method based on air pressure measuring according to claim 6, wherein in said functional DNA sensing system, quantitative analysis method based on air pressure measuring, comprising the following steps: (1)selecting appropriate aptamer according to the target molecules, add a sequence complementary to capture probe to the aptamer; (2) designing suitable detection probe sequence according to the sequence of aptamer, allowing when there are not target molecular, the detection probe can combine onto the aptamer strand through the interaction between bases, while when the target molecules exist, the detection probes can be replaced by the target molecule; (3) connecting the capture probe onto the surface of magnetic bead by biological or chemical conjugation method, which comprising the decarboxylation reaction or biotin avidin interaction, adding detection DNA and aptamer, form sandwich hybridization structure of functionalization DNA on the surface of the magnetic beads; (4) adding target in DNA sandwich hybridization system , target combined to aptamer, thus the detection probe on three-strand-structure be replaced out; (5) using magnet to enrich magnetic beads, and put the supernatant into a reaction tube, add a solution phase substrates, signal amplification molecules act with the solution phase substrates, then produce signal molecules which can be read by the air pressure meter. (6) recording test results according to air pressure meter readings.

10. The quantitative analysis method based on air pressure measuring according to claim 7, wherein said detection probe combined with signal amplification mocules which can catalyze substrate to generate large amount of gas molecules.

11. The quantitative analysis method based on air pressure measuring according to claim 9, wherein the combination of the aptamer and the target molecules, enables the target molecules to compete and replace to release the detection probe conjugated with signal amplification molecules (enzyme or nanoparticles), and realize follow-up signal amplification.


sequence listing
<110>XIAMEN UNIVERSITY
<120>A quantitative analysis method based on air pressure messuring
<160>NUMBER OF SEQ ID NOS:18


<210>SEQ ID NO 1
<211>length:56
<212>TYPE:DNA
<213>ORGANISM: Artificial Sequence

<400>SEQUENCE:1
ACTCATCTGT GAATCTCGGG AGACAAGGAT AAATCCTTCA ATGAAGTGGG TCTCCC 56


<210>SEQ ID NO 2
<211>length:17
<212>TYPE: DNA
<213> ORGANISM: Artificial Sequence

<400>SEQUENCE:2
AAAATCACAG ATGAGT 17


<210>SEQ ID NO 3
<211>length:16
<212>TYPE: DNA
<213> ORGANISM: Artificial Sequence

<400>SEQUENCE:3
AAAAGTCTCC CGAGAT 16


<210>SEQ ID NO 4
<211>length:44
<212>TYPE: DNA
<213> ORGANISM: Artificial Sequence

<400>SEQUENCE:4
ACTCATCTGT GAAGAGAACC TGGGGGAGTA TTGCGGAGGA AGGT 44
80


<210>SEQ ID NO 5
<211>length:16
<212>TYPE: DNA
<213> ORGANISM: Artificial Sequence

<400>SEQUENCE:5
AAAATCACAG ATGAGT 16


<210>SEQ ID NO 6
<211>length:16
<212>TYPE: DNA
<213> ORGANISM: Artificial Sequence

<400>SEQUENCE:6
AAAACCCAGG TTCTCT 16


<210>SEQ ID NO 7
<211>length:50

<212>TYPE: DNA
<213> ORGANISM: Artificial Sequence

<400>SEQUENCE:7
CTACAAAAAC ACATACTCAC TATAGGAAGA GATGATCCAC ATTTTCACAG          50


<210>SEQ ID NO 8
<211>length:28
<212>TYPE: DNA
<213> ORGANISM: Artificial Sequence

<400>SEQUENCE:8
CATCTCTGAA GTAGCGCCGC CGTATAGT          28


<210>SEQ ID NO 9
<211>length:14
<212>TYPE: DNA
<213> ORGANISM: Artificial Sequence

<400>SEQUENCE:9
CTGTGAAAAT GTGG          14


<210>SEQ ID NO 10
<211>length:14
<212>TYPE: DNA
<213> ORGANISM: Artificial Sequence

<400>SEQUENCE:10
ATGTGTTTTT GTAG          14


<210>SEQ ID NO 11
<211>length:54
<212>TYPE: DNA
<213> ORGANISM: Artificial Sequence

<400>SEQUENCE:11
CAGTGTCTGT GAGTATGCGA TCGGGTGTGG GTGGCGTAAA GGGAGCATCG GACA          54


<210>SEQ ID NO 12
<211>length:11
<212>TYPE: DNA
<213> ORGANISM: Artificial Sequence

<400>SEQUENCE:12
CACAGACACT G          11


<210>SEQ ID NO 13
<211>length:11
<212>TYPE: DNA
<213> ORGANISM: Artificial Sequence

<400>SEQUENCE:13
CCGATCGCAT A          11


<210>SEQ ID NO 14
<211>length:24

<212>TYPE: DNA
<213> ORGANISM: Artificial Sequence

<400>SEQUENCE:14
TCACAGATGA GTAAAAAAAA AAAA          24

<210>SEQ ID NO 15
<211>length:62
<212>TYPE: DNA
<213> ORGANISM: Artificial Sequence

<400>SEQUENCE:15
TTTTTTACTC ATCTGTGAAT CTCGGGAGAC AAGGATAAAT CCTTCAATGA AGTGGGTCTC
CC                                                          62

<210>SEQ ID NO 16
<211>length:24
<212>TYPE: DNA
<213> ORGANISM: Artificial Sequence

<400>SEQUENCE:16
AAAAAAAAA AAGTCTCCCG AGAT           24

<210>SEQ ID NO 17
<211>length:50
<212>TYPE: DNA
<213> ORGANISM: Artificial Sequence

<400>SEQUENCE:17
TTTTTTACTC ATCTGTGAAG AGAACCTGGG GGAGTATTGC GGAGGAAGGT    50
78

<210>SEQ ID NO 18
<211>length:24
<212>TYPE: DNA
<213> ORGANISM: Artificial Sequence

<400>SEQUENCE:18
AAAAAAAAA AACCCAGGTT CTCT                            24